# EUROPÄISCHE PATENTSCHRIFT

(11) **EP 1 327 005 B1**
(45) Veröffentlichungstag und Bekanntmachung des Hinweises auf die Patenterteilung: **13.06.2012**
(21) Anmeldenummer: 01986324.0
(22) Anmeldetag: 02.10.2001
(51) Int. Cl.: C12Q 1/68

(54) **VERFAHREN ZUR SELEKTION HOCHAFFIN AN EIN TARGET BINDENDER NUKLEINSÄUREN**
METHOD FOR SELECTING NUCLEIC ACIDS THAT BOND WITH HIGH-AFFINITY TO A TARGET
PROCEDE DE SELECTION D'ACIDES NUCLEIQUES SE LIANT A UNE CIBLE AVEC UNE AFFINITE ELEVEE

(30) Priorität: 02.10.2000 DE 10049074
(43) Veröffentlichungstag der Anmeldung: 16.07.2003
(73) Patentinhaber: AptaRes AG, 14943 Luckenwalde (DE)
(72) Erfinder: Kage, Andreas, Dr., 12007 Berlin (DE)
(74) Vertreter: Jungblut, Bernhard Jakob
(86) Internationale Anmeldenummer: PCT/DE2001/003817
(87) Internationale Veröffentlichungsnummer: WO 2002/029093

(56) Entgegenhaltungen:
- WO-A-91/19813
- US-A- 5 004 547
- FAMULOK M ET AL: "IN VITRO SELECTION OF SPECIFIC LIGAND-BINDING NUCLEIC ACIDS**" ANGEWANDTE CHEMIE. INTERNATIONAL EDITION, VERLAG CHEMIE. WEINHEIM, DE, Bd. 31, Nr. 8, 1. August 1992 (1992-08-01), Seiten 979-988, XP000298700 ISSN: 0570-0833
- PIERROU S ET AL: "SELECTION OF HIGH-AFFINITY BINDING SITES FOR SEQUENCE-SPECIFIC, DNABINDING PROTEINS FROM RANDOM SEQUENCE OLIGONUCLEOTIDES" ANALYTICAL BIOCHEMISTRY, ACADEMIC PRESS, SAN DIEGO, CA, US, Bd. 229, Nr. 1, 20. Juli 1995 (1995-07-20), Seiten 99-105, XP000524716 ISSN: 0003-2697
- THIESEN H J ET AL: "TARGET DETECTION ASSAY (TDA): A VERSATILE PROCEDURE TO DETERMINE DNA BINDING SITES AS DEMONSTRATED ON SP1 PROTEIN" NUCLEIC ACIDS RESEARCH, OXFORD UNIVERSITY PRESS, SURREY, GB, Bd. 18, Nr. 11, 11. Juni 1990 (1990-06-11), Seiten 3203-3209, XP000132496 ISSN: 0305-1048

## Beschreibung

### Gebiet der Erfindung

Die Erfindung betrifft ein Verfahren zur Selektion hochaffin an ein Target bindender Nukleinsäuren, wobei ein Gemisch von Nukleinsäuren mit einem oder mehreren definierten Targetmolekülen kontaktiert wird, wobei an dem Targetmolekül bindende Nukleinsäuren immobilisiert werden und wobei die an das Targetmolekül gebundenen Nukleinsäuren, nach Entfernung nicht gebundener Nukleinsäuren, von den Targetmolekülen desorbiert werden.

Als Nukleinsäuren sind Poly- oder Oligonukleotide mit einer Nukleotidzahl von 5 bis 200, insbesondere 20 bis 200, bezeichnet. Es kann sich dabei um DNA, RNA oder PNA handeln. Insbesondere können die Nukleinsäuren chemisch derivatisiert, beispielsweise durch 2' und/oder 5 Substitution, und/oder mit Reportermolekülen (Moleküle, welche einen Nachweis mit üblichen Detektionsmethoden erlauben) versehen sein. Die Nukleinsäure kann einzel- oder doppelsträngig sein. Ein Targetmolekül kann grundsätzlich beliebiger Art sein, sofern es sich nicht seinerseits um eine Nukleinsäure handelt, welche mit der der damit kontaktierten Nukleinsäure Crick/Watson Basenpaar-Bindungen eingeht. Beispiele für Targetmoleküle sind: Peptide, Proteine, Enzyme, Oligosacharide, Polysaccharide, Nukleinsäuren, welche nicht Crick/Watson Bindungen eingehen, Lipide, aber auch Hormone und andere organische Verbindungen (Pheromone). Targets können auch Zellbestandteile und ganze Zellen sein, ebenso wie vollständige Viren und Bakterien. An nicht-Nukleinsäuren bindende Nukleinsäuren werden auch als Aptamere bezeichnet, aber auch Nukleinsäuren, welche mit anderen Nukleinsäuren nicht-Crick/Watson Bindungen eingehen. Letztgenannte Aptamere können beispielsweise eingesetzt werden zur Erkennung von bestimmten Gendefekten und/oder Deletionen in Genen. Der Begriff der Bindung meint im Rahmen der Erfindung nicht-kovalente Bindungen. Der Begriff der Affinität bezieht sich im Rahmen der Erfindung auf die Bindungsfestigkeit innerhalb von Komplexen der Antigen/Antikörper Art. Die Bindungsfestigkeit ist durch die Affinitätskonstante quantifizierbar, welche definiert ist nach dem Massenwir- kungsgesetz. Im Rahmen der Erfindung bezieht sich der Begriff der Affinität allerdings nicht nur auf Komplexe mit einer Bindungsstelle, sondern auch auf Komplexe mit mehreren Bindungsstellen, i.e. umfaßt auch den Begriff der Avidität. Die Avidität ergibt sich aus der Anzahl und der Bindungsstärke jeder Bindungsstelle bei mehrvalenten Antikörper/Antigen Komplexen. Als Amplifikation wird jede enzymatisch vermittelte Reaktion verstanden, die der Replikation einer Nukleinsäure dient, beispielsweise die PCR.

### Hintergrund der Erfindung und Stand der Technik

Nukleinsäuren dienen in natürlichen Organismen in erster Linie zur Codierung von in einer Zelle zu exprimierenden Proteinen und dergleichen. Hierfür ist die Primärstruktur der Nukleinsäuren, i.e. die Sequenz maßgeblich. Unabhängig hiervon können Nukleinsäuren jedoch auch Antikörper/Antigen Komplexe mit in einer Zelle vorliegenden nicht-Nukleinsäuren oder, in Sonderfällen, nicht-Crick/Watson Bindungen mit anderen Nukleinsäuren eingehen. Ob eine solche Bindung stattfinden kann, hängt dabei nicht nur von der Primärstruktur ab, sondern auch von der in einer Lösung entstehenden Sekundär-, Tertiar und Quarternärstruktur bei einer definierten Sequenz (dreidimensionale Struktur) ab. Die Affinität der Nukleinsäure zu dem Targetmolekül hängt daher letztendlich davon ab, ob die Nukleinsäure - neben der rein chemischen Bindungsfähigkeit - in sterischer Hinsicht im Bereich der Bindungsstelle oder der Bindungsstellen des Targetmoleküls "paßt", entsprechend der Verhältnisse bei klassischen Antikörper/Antigen Komplexen. Passende Nukleinsäuren können also die Funktion eines Antikörpers oder Antigens ausüben. Solche Aptamere sind in aller Regel nicht-natürliche Nukleinsäuren und können gleichsam "maßgeschneidert" werden für ein Targetmolekül. Für die Maßschneiderung gibt es grundsätzlich zwei Ansätze. Der erste Ansatz besteht in der Berechnung einer geeigneten Sequenz und/oder Derivatisierung für die Nukleinsäure nach Maßgabe der genau bekannten Struktur, einschließlich Bindungsstellen und Tertiärstruktur, des Targetmoleküls. Dies ist nicht nur extrem aufwendig; in Fällen, in welchen die Struktur des Targetmoleküls nicht hinreichend bekannt ist, ist dieser Ansatz sogar unmöglich. Der zweite Ansatz besteht in der Isolierung des Targetmoleküls und in der Kontaktierung einer Mischung aus prospektiven Nukleinsäuren mit dem isolierten (und meist immobilisierten) Targetmolekül, wobei hochaffin bindende Nukleinsäuren von den weniger affin oder gar nicht bindenden Nukleinsäuren abgetrennt werden. Bei den Mischungen mit Nukleinsäuren handelt es sich typischerweise um Nukleinsäurenbibliotheken, welche beispielsweise im Wege der kombinatorischen Chemie hergestellt wurden. Eine Nukleinsäurebibliothek enthält eine Vielzahl voneinander unterschiedlicher Nukleinsäuren, wobei zumindest in einem Teilsequenzbereich eine Randomisierung (mit natürlichen und/oder nicht-natürlichen Nukleotiden) eingerichtet ist. Es kann, muß aber nicht, ein konservierter Sequenzbereich vorgesehen sein. Randomisierung in n Positionen mit m verschiedenen Nukleotiden führt zu einer Bibliothek mit n^{m} Elementen. Die selektierten hochaffinen Nukleinsäuren sind für eine Vielzahl von Anwendungen geeignet.

So können die Nukleinsäuren im Rahmen von Tests auf Vorliegen oder Nichtvorliegen der für die Nukleinsäure spezifischen Targetmoleküle in einer Testlösung und/oder in einer Zelle bzw. Gewebe verwendet werden. Dann empfiehlt sich die Anwesenheit eines Reportermoleküls üblichen Aufbaus in der Nukleinsäure zur leichten meßtechnischen Identifizierbarkeit. Solche Tests können in der Diagnostik, beispielsweise der Diagnostik auf onkogene Mutanten oder Markersubstanzen, welche bei bestimmten physiologischen Fehlfunktionen entstehen, eingesetzt werden. Es versteht sich, daß die betreffende Nukleinsäure dann nicht nur die onkogene Mutante selektiv "erkennen" muß, sondern an die natürliche Variante demgegenüber nicht binden darf, i.e. diskriminieren muß zwischen onkogenem Expressionsprodukt und natürlichem Expressionsprodukt. Dies läßt sich leicht nach der Selektion an das onkogene Expressionprodukt bindender Nukleinsäuren durchführen, nämlich durch die subsequente Selektion von an das natürliche Expressionsprodukt nicht bindender Nukleinsäuren aus den zuvor selektierten Nukleinsäuren.

Selektierte Nukleinsäuren können auch zur Abtrennung von Targetmolekülen aus einer Lösung verwendet werden, beispielsweise im Rahmen üblicher Säulen- oder Geltrennungsverfahren. Dann empfiehlt es sich, die Nukleinsäure immobilisierbar zu gestalten und in dem Trennungsverfahren zu immobilisieren.

Selektierte Nukleinsäuren können aber auch dazu eingesetzt werden, um physiologische Funktionen zu modulieren, i.e. zu hemmen, zu induzieren oder zu verstärken. Solche Nukleinsäuren haben daher auch Verwendung in pharmazeutischen Zusammensetzungen. Selektierte Nukleinsäuren müssen natürlich ansonsten physiologisch unbedenklich sein zur Vermeidung von Nebenwirkungen. Der Vorteil des Einsatzes von Nukleinsäuren anstelle von beispielsweise Peptiden liegt darin, daß die Identifizierung bzw. Selektion geeigneter Nukleinsäuren erheblich einfacher als im Falle der Peptide und Proteine ist wegen der vergleichsweise einfachen Herstellbarkeit von Nukleinsäurebibliotheken gegenüber Protein- oder Peptidbibliotheken.

Ein bekanntes Verfahren zur Selektion hochaffin an ein Targetmolekül bindender Nukleinsäuren ist als das SE-LEX Verfahren (Systematic Evolution of Ligands by EXponential Enrichment) bekannt. Verschiedene Varianten und Anwendungsbereich sind beispielsweise beschrieben in den Literaturstellen US-A-5,712,375, US-A-5,864,026, US-A-5,789,157, US-A-5,475,096, US-A-5, 861, 254, US-A-5, 595, 877, US-A-5, 817, 785. Bei dem insofern bekannten Verfahren werden grundsätzlich approximativ in einer Mehrzahl von Zyklen diejenigen Nukleinsäuren separiert, welche mit hoher bzw. immer höherer Affinität binden. Dabei muß im Rahmem jedes Zyklus die selektierte Gruppe an Nukleinsäuren amplifiziert werden. Die Loslösung bindender Nukleinsäuren vom Target in jedem Zyklus erfolgt durch spezifische Verdrängung. Dieses Verfahren hat mehrere Nachteile. Zunächst ist nachteilig, daß aufgrund der erforderlichen Zyklenzahl eine recht große Menge sowohl an Nukleinsäuren als auch an Targetmolekülen erforderlich ist. Weiterhin werden bei der Verdrängungsverfahrenstufe mehr (gebundene) Nukleinsäuren geringerer Affinität aus der Bindung verdrängt als Nukleinsäuren höherer Affinität, wodurch der Mengenunterschied zu Lasten der höheraffinen Nukleinsäuren im Rahmen der Amplifikationsverfahrensstufe verstärkt wird. Der Mengenunterschied wird zudem noch weiter dadurch verstärkt, daß bei höherer Affinität die Bindung der losgelösten Nukleinsäuren an die zur Loslösung eingesetzten Liganden vergleichsweise stärker ist und die höheraffinen Nukleinsäuren dadurch schlechter für die Amplifikation zugänglich sind. Nachteilig ist weiterhin, daß mit zunehmender Affinität der Nukleinsäuren eine logarithmisch ansteigende Konzentration des zur Loslösung verwendeten Liganden erforderlich wird. Die erreichbare Affinität ist somit vom Löslichkeitsprodukt der eingesetzten Liganden limitiert. Schließlich ist es nachteilig, in mehrern Zyklen arbeiten zu müssen zur wiederholten Abtrennung bzw. Selektion in einem Vorzyklus selektierter Nukleinsäuren.

Im Bereich der Trennung von nicht-Nukleinsäuren ist die Affinitätschromatographie, insbesondere als Säulenchromatographie (Fest-/Flüssigphase) wohl bekannt. Es handelt sich hierbei um ein Verfahren zur Isolierung oder Anreicherung, bis zu 10⁵-fach und mehr, von beispielsweise Proteinen. Ein Ligand der anzureichernden Substanz wird dabei an einer chromatographischen Matrix immobilisiert. Hochaffine Verbindungen werden zuerst, i.e. säuleneingangsseitig, gebunden. Stromabwärts werden weniger affine Verbindungen gebunden, sofern die Menge der affinen Verbindungen, bezogen auf jeweilige konkrete Verbindung, niedriger als die Menge der Liganden in der Säule ist. Gering affine oder nicht affine Verbindungen laufen durch und werden so von den affinen Verbindungen getrennt. Gebundene, i.e. affine Verbindungen werden dann eluiert und weiter verwendet. Bei unspezifischen Desorptionsverfahren, beispielsweise physikochemischen oder thermischen Verfahren, findet ein Vermengung unterschiedlich hoch affiner (gebundener) Verbindungen statt. Bei der Desorption mit Liganden der gebundenen Verbindungen (Verdrängungsdesorption) ist eine sehr große molare Menge des Liganden zur Loslösung insbesondere der hochaffinen Verbindungen notwendig.

### Technisches Problem der Erfindung

Der Erfindung liegt das technische Problem zugrunde, ein Verfahren zur Selektion hochaffin an ein Targetmolekül bindender Nukleinsäuren anzugeben, welches mit weniger Aufwand hochaffine Nukleinsäuren mit sehr geringer Varietät in den selektierten Nukleinsäuren liefert.

### Grundzüge der Erfindung

Zur Lösung dieses technischen Problems lehrt die Erfindung ein Verfahren zur Selektion hochaffin an ein Targetmolekül bindender Nukleinsäuren aus einem Gemisch von Nukleinsäuren mit den folgenden Verfahrensschritten: a) eine Säule wird (innenseitig) mit den Targetmolekülen beladen und die Targetmoleküle werden in der Säule immobilisiert, b) das Gemisch an Nukleinsäuren wird einem ersten Ende der Säule aufgegeben, wobei durch die Säule ein definierter Volumenstrom an Trägersubstanz, laufend vom ersten Ende zum zweiten Ende der Säule, eingerichtet ist, c) die Nukleinsäuren werden mit abnehmender Affinität der Nukleinsäuren zum Targetmolekül in zunehmendem Abstand vom ersten Ende der Säule gebunden und immobilisiert, d) der Volumenstrom an Trägersubstanz durch die Säule wird nach einer definierten Laufzeit beendet, e) die Säule wird durch eine Mehrzahl von Teilungen in Säulensegmente aufgetrennt, wobei jedem Segment eine Laufwegkoordinate zugeordnet wird, f) aus zumindest einem Segment werden die immobilisierten Nukleinsäuren unspezifisch desorbiert und unter Zuordnung der dem Segment zugeordneten Laufwegkoordinate gewonnen. Der Ausdruck innenseitig der Säule meint innerhalb eines Lumens in aller Allgemeinheit. Der Ausdruck der Säule soll im Rahmen der Erfindung alle Arten fester Trägersysteme umfassen, i.e. auch nicht vollständig umschlossene Trägersysteme sind möglich. Im Rahmen der Erfindung kann im Grenzfall lediglich ein Säulensegment vorgesehen sein.

Die Immobilisierung der Targetmoleküle kann nach den üblichen Methoden der Säulenchromatographie erfolgen. Es kann eine Spezies an Targetmolekülen eingesetzt werden, es können aber auch mehrere verschiedene definierte Spezies, in homogener Verteilung oder nach der Laufwegkoordinate geordnet, innerhalb der Säule, eingesetzt werden. Die Art der Immobilisierung hängt im Einzelfall von der Struktur und/oder den Eigenschaften des Targetmoleküls ab und kann vom Fachmann unschwer aus dem Stand der Technik ausgewählt werden. Als Säule ist jedes mechanisches Konstrukt bezeichnet, welches ein Lumen mit zwei Enden aufweist. Als Strukturmaterial kommen alle für Säulen üblichen Materialien, wie Metalle, Glas und/oder Kunststoffe in Frage. Die Säule kann innenseitig mit einer das Targetmolekül bindenden Matrix versehen sein und/oder das Strukturmaterial kann zur direkten Bindung der Targetmoleküle geeignet oder vorbereitet sein. Ein Gemisch aus Nukleinsäuren bezeichnet Nukleinsäurebibliotheken mit einer Anzahl von typischerweise 10⁶ bis 10²²/Mol, insbesondere 10¹⁰ bis 10²¹/Mol, voneinander verschiedener Nukleinsäurenspezies. In der auf die Säule aufgetragenen Bibliothek ist jede Nukleinsäurespezies statistisch beispielsweise mit 10 bis 10¹⁷, insbesondere 100 bis 10¹³, Molekülen vertreten. Die Trägersubstanz ist üblicherweise eine Flüssigkeit, in welcher die Nukleinsäurebibliothek löslich und stabil ist. Hierfür kommen alle für Nukleinsäurebibliotheken üblichen Puffer und dergleichen in Frage. Der Volumenstrom an Trägersubstanz kann vor Auftrag der Nukleinsäurebibliothek eingestellt werden. Dann wird die Nukleinsäurebibliothek säuleneingangsseitig dem Trägersub- stanzstrom zugegeben. Die Nukleinsäurebibliothek kann aber auch unmittelbar aufgegeben werden. Nach einer Laufzeit, welche durch den Aufbau der Säule und den eingestellten Volumenstrom bestimmt ist, tritt der "Pfropfen", welcher durch die Nukleinsäurebibliothek aufgegeben wurde, säulenausgangsseitig wieder aus (verbreitert durch Faltung mit der Diffusion), wobei gebundene Nukleinsäuren aus dem "Pfropfen" abgetrennt und in der Säule immobilisiert wurden. Zweckmäßigerweise wird der Volumenstrom durch die Säule wenig- bzw. nicht-turbulent, vorzugsweise laminar, eingestellt (Summe der Beschleunigungsvektoren der Trägersubstanz über.das Säulenvolumen, insbesondere über den Säulenquerschnitt, minimal, idealerweise 0). Die Gesamtanzahl der Targetmoleküle (einer Targetmolekülspezies) in der Säule beträgt typischerweise das 10²- bis 10¹⁶-fache, insbesondere das 10³- bis 10¹⁵-fache, der Anzahl an Nukleinsäuremolekülen einer einzelnen Spezies in der aufgetragenen Nukleinsäurebibliothek. Die Bindung der Nukleinsäuren an die Targetmoleküle erfolgt vorzugsweise unter Bedingungen, welche einem späteren Einsatz der Nukleinsäuren entsprechen, i.e. beispielsweise in einem Puffer bzw. einer Trägersubstanz, welche entsprechend abgestimmt ist hinsichtlich Temperatur, Ionenstärke, pH-Wert und Pufferbedingungen. Die Trägersubstanz sowie das Lösungsmittel der Nukleinsäurenbibliothek sind dann entsprechend hinsichtlich derer Komponenten auszuwählen. Die Teilung der Säule in eine Mehrzahl von Säulensegmente kann beispielsweise durch Zerschneiden der Säule erfolgen, wobei die Schnitte vorzugsweise orthogonal zum Volumenstromvektor erfolgen. Die unspezifische Desorption kann durch Eluierung mit einem hinreichend starken Liganden durch Verdrängung, Komplexierung, Modifikation und/oder Zerstörung des Targetmoleküls, physikochemisch oder thermisch erfolgen. Auch mechanische Verfahren, beispielsweise Ultraschall, können zur Desorption oder zur Verstärkung der Desorption eingesetzt werden. Es können auch Kombinationen der vorstehenden Desorptionsverfahren angewandt werden. Es versteht sich, daß die Nukleinsäuren von dem verwendeten Desorptionsverfahren nicht zersetzt werden dürfen.

Die Erfindung beruht auf der Erkenntnis, daß eine Nukleinsäurebibliothek sich analog einer Proteinmischung mittels der Affinitätschromatographie räumlich nach Maßgabe der Affinität zum Targetmolekül trennen läßt. Die Erfindung beruht weiterhin auf der Erkenntnis, daß eine mittels unspezifischer Desorption sich einstellende Vermengung desorbierender Nukleinsäuren verschiedener Affinität dadurch vermeidbar ist, daß vor der unspezifischen Desorption die Säule mit den darin gebundenen Nukleinsäuren gleichsam in Affinitätsabschnitte aufgeteilt und die in den so erhaltenen Affinitätsabschnitten bzw. Säulensegmenten gebundenen Nukleinsäuren sich leicht und ohne störende Ligandenkoppelungen beispielsweise im Rahmen einer PCR oder RT-PCR unspezifisch desorbieren und ebenso unspezifisch amplifizieren lassen. Subsequente Seletionsartefakte zu Lasten höheraffiner Nukleinsäuren werden vermieden. Ebensowenig sind Liganden, insbesondere hohe Konzentrationen an Liganden, zur Desorption erforderlich. Schließlich stehen praktisch alle gebundenen und dann desorbierten Nukleinsäuremoleküle für eine Amplifikation zur Verfügung. Dies erlaubt es, mit geringen Nukleinsäurekonzentrationen zu arbeiten. Es ist grundsätzlich bereits ausreichend, wenn in der Nukleinsäurebibliothek jede Spezies im statistischen Mittel mit einem Molekül vertreten ist. Wenn die Anzahl der Targetmoleküle in einem Säulensegment statistisch 1 beträgt, dann können sogar einzelne Nukleinsäurespezies nach ihrer Affinität zum Targetmolekül getrennt werden.

Ein besonderer Vorzug der Erfindung ist folgend erläutert. Die Auftrennung der Nukleinsäuren nach Affinität führt dazu, daß Nukleinsäuren in einem Säulensegment eine ähnlich Affinität bei unterschiedlicher Spezifität (unterschiedliche Regionen des Targets werden gebunden bzw. sind Bindungsstellen für die Nukleinsäuren) aufweisen. So erhaltene Nukleinsäuregemische können in Anwendungen eingesetzt werden, die bisher oligoklonalen Antiseren vorbehalten sind. So ist beispielsweise eine quantitative Bestimmung von Targetmolekülen durch Einsatz des Nukleinsäuregemisches im Rahmen eines Bindungskits möglich. Dabei kann das Nukleinsäurengemisch aus einem Säulensegment zum spezifischen Immobilisieren bestimmter Targetmoleküle (Capture Eigenschaft) und/oder zur spezifischen Detektion der immobilisierten Targetmoleküle eingesetzt werden.

Mit dem erfindungsgemäßen Verfahren isolierte Nukleinsäuren bzw. hergestellte Nukleinsäuregemische (kurz Nukleinsäuren) lassen sich vielfältig verwenden. Für eine jeweilige konkrete Anwendung ist es lediglich notwendig, nach Maßgabe der Anwendung ausgewählte Targetmoleküle in der Säule zu immobilisieren. So ist es beispielsweise möglich, für eine Erkrankung charakteristische Markersubstanzen zu identifizieren, mit dem erfindungsgemäßen Verfahren hochaffin daran bindende Nukleinsäuren zu bestimmen und die so selektierten Nukleinsäuren als Hauptkomponente eines Testkits zur Untersuchung auf die Markersubstanz bzw. auf das Vorliegen der Erkrankung oder das Risiko der Erkrankung einzusetzen. Solche Testkits können selbstverständlich auch zur Therapiekontrolle eingesetzt werden. Die Nukleinsäuren können auch zur Herstellung von pharmazeutischen Zusammensetzungen verwendet werden, beispielsweise wenn eine Hemmung der Markersubstanz zu einer Verringerung oder Verhinderung des Krankheitsbildes führt. Es ist aber auch möglich, durch Auswahl der Targetmoleküle Nukleinsäuren zu selektieren und als pharmazeutische Zusammensetzung zu verwenden, welche die Bildung von im Krankheitsfall fehlenden Stoffen in einem Organismus stimulieren. Durch Auswahl geeigneter Targetmoleküle lassen sich beispielsweise auch Nukleinsäuren finden, welche als Wirkstoffe die Differenzierung und/oder Stimulation oder Suppression von isolierten Zellen (beispielsweise Blutzellen, wie T-Zellen, Granulozyten, Monozyten oder Thrombozyten) beeinflussen. Gleiches kann für Zellen in einem Verbund (Gewebe) u.a. im Rahmen des Tissue Engineering bewirkt werden. Schließlich können erfindungsgemäß selektierte Nukleinsäuren beispielsweise in einer Affinitätsmatrix als immobilisierte Phase eingesetzt werden (z.B. Pheresetechnologien) oder zur spezifischen Desorption von Substanzen von einer Affinitätsmatrix verwendet werden.

### Bevorzugte Ausführungsformen der Erfindung

Grundsätzlich reicht es aus, wenn ein Segment, welchem eine gewünschte Affinität zugeordnet ist, (isoliert) zur Desorption weiterverarbeitet wird. Dies setzt allerdings - außer bei maximaler Affinität, wobei das, bezogen auf den Volumenstromvektor, erste Säulensegment weiter verarbeitet wird - eine Vorstellung über die Affinitätsverteilung bei der aufgetragenen Nukleinsäurebibliothek voraus. Bevorzugt ist es daher in der Regel, daß die immobilisierten Nukleinsäuren aus jedem Segment separat desorbiert und gewonnen werden unter jeweiliger Zuordnung der Laufwegkoordinaten jeden Segments zu den daraus gewonnenen Nukleinsäuren.

Grundsätzlich ist jede Art der Desorption möglich. Vorzugsweise wird die unspezifische Desorption mittels üblicher physikochemischer oder thermischer Verfahren durchgeführt wird. Thermische Desorption erfolgt durch Erwärmung des Säulensegments bzw. der darin enthaltenen Lösung. Die Erwärmung kann beispielsweise durch elektrische Beheizung oder Einstrahlung von Mikrowellen oder IR erfolgen. Insbesondere sind die Erwärmungstechniken aus der PCR Technologie geeignet. Neben der Amplifikation von Nukleinsäuren bzw. Aptameren mittels Polymerase können selbstverständlich auch andere Amplifikationsverfahren, wie beispielsweise mittels Ligase, angewandt werden. Die unspezifische Desorption kann durch chemische Modifikation des Targets unterstützt werden, z.B. Oxidation durch Natriumperjodat oder dergleichen, oder durch unspezifische Komplexbildung, z.B. mittels Borat oder dergleichen zur Blockierung von cis-trans-Diolbindungen in Kohlenhydraten.

Daher ist es bevorzugt, wenn die unspezifische Desorption durch thermische Desorption in einer, vorzugsweise verlängerten, Hochtemperaturphase einer PCR oder RT-PCR durchgeführt wird. Hierbei wird ein Synergieeffekt erzielt, da in aller Regel, insbesondere beim Arbeiten mit Nukleinsäurebibliotheken mit niedrigen Konzentrationen der Nukleinsäurespezies, eine Amplifizierung ohnehin erforderlich ist. Zur Erhöhung der Ausbeute wird beispielsweise mit 5 bis 60, vorzugsweise mit 20 bis 60, höchstvorzugsweise mit 45 bis 55, Zyklen gearbeitet. Im Rahmen der Amplifikation kann mit mindestens einem markierten Primer gearbeitet werden. Der Primer kann mindestens eine Endonukleaseschnittstelle aufweisen. Eine solche Schnittstelle dient beispielsweise dazu, das Amplifikat von größeren Bereichen der Primersequenz zu befreien. Nukleotidbausteine, sei es im Primer oder bei den zu selektierenden Nukleinsäuren, können beispielsweise mittels Fluoreszenzfarbstoffen markiert sein. Als Fluoreszenzfarbstoffe seien beispielsweise genannt: Alexa^{®} Fluor 488, Fluor 532, Fluor 546, Fluor 568, Fluor 594, Oregon Green 488, Fluorescein, Rhodamine 6G, Tetramethylrhodamine, Rhodamine B und Texas Red. Das Amplifikat kann auch durch zwei verschiedene chemische Modifikationen an verschiedenen Enden markiert sein, sofern die bei der Modifikation eingeführten Gruppen geeignet sind, als Liganden jeweils an einer unterschiedlichen Affinitätsmatrix gebunden werden zu können.

Es empfiehlt sich, zur sicheren Abtrennung nichtaffiner oder niedriger-affiner Nukleinsäuren zwischen geeigneten Verfahrenstufen Waschverfahrenschritte einzufügen. Insbesondere ist es bevorzugt, wenn zwischen den Verfahrensschritten d) und e) zumindest ein Waschverfahrensschritt durchgeführt wird. Zum Waschen ist beispielsweise das Lösungsmittel bzw. das Medium der Nukleinsäurebibliothek bzw. die Trägersubstanz geeignet.

Bevorzugt ist es, wenn die innenseitige Belegung der Säule mit Targetmolekülen und deren Immobilisierung mittels kovalenter Bindung, vorzugsweise nach Aktivierung mit chemisch hochreaktiven Gruppen (beispielsweise Tresylchlorid, Cyanbromid und/oder Periodat) oder über bifunktionale Spacerverbindungen nach Modifikation mit chemisch wenig reaktiven Gruppen (beispielsweise Amin, Hydroxy, Keto und/oder Carboxyl), durchgeführt wird. Beispiele für Spacergerüste geeigneter Spacerverbindungen sind: substituierte und unsubstituierte C2 -C10 Alkylgruppen, substituierte und unsubstituierte C2 -C10 Alkenylgruppen, substituierte und unsubstituierte C2 -C10 Alkynylgruppen, substituierte und unsubstituierte C4 -C7 Carbocylo alkylgruppen, substituierte und unsubstituierte C4 -C7 Carbocylo alkenylgruppen, substituierte und unsubstituierte C7 -C14 Aralkylgruppen, ein heterocyclisches Molekül mit Heteroatomen ausgewählt aus Stickstoff, Sauerstoff, Schwefel, wobei besagte Substitutionen bestehen können aus Alkyl, Alkenyl, Alkynyl, Alkoxy, Thiol, Thioalkoxy, Hydroxyl, Aryl, Benzyl, Phenyl, Nitro, Halogen, Äthergruppen mit 2 bis 10 Kohlenstoffatomen und 1 bis 4 Sauerstoff oder Schwefel Atomen, Polyalkyl glycol, Halogen, Hydroxyl, Thiol, Keto, Carboxyl, Amide, Ätherverbindungen, Thioäther, Arnidinederivaten, Guanidinederivaten, Glutamylderivaten, Nitrat(ONO₂), Nitro (NO₂), Nitril, Trifluoromethyl (-CF₃), Trifluoromethoxy (-OCF₃), O-Alkyl, S-Alkyl, NH-Alkyl, N-Dialkyl, O-Aralkyl, S-Aralkyl, NH-Aralkyl, Amino, Azido (N3), Hydrazino (NHNH₂), Hydroxylamino (ONH₂), Sulfoxide (SO), Sulfone (SO₂), Sulfide (S-), Disulfide (S-S), Silyl bestehen kann. Spacerverbindungen sind typischerweise bifunktional, wobei die Funktionalitäten gleich oder verschieden sein können und beispielsweise ausgewählt sind aus der Gruppe bestehend aus "N-Hydroxysuccinimid und Hydrazide".

Zur Verringerung der Varietät innerhalb der aus einem Säulensegment erhältlichen Nukleinsäuren ist es bevorzugt, wenn jedes Säulensegment im statistischen Mittel 0,1 bis 10³, vorzugsweise 1 bis 10², höchstvorzugsweise 1 bis 10, Targetmoleküle enthält. Hierauf abgestimmt kann die Nukleinsäurebibliothek, wie aufgetragen, im statistischen Mittel 0,1 bis 10³, vorzugsweise 1 bis 10², höchstvorzugsweise 1 bis 10, Nukleinsäuremoleküle einer Spezies enthalten.

Für das Strukturmaterial der Säule kommen grundsätzlich alle beispielsweise aus der Affinitätschromatographie bekannten Werkstoffe in Frage. Hierzu gehören Säulen aus Kieselgel oder Polymere, wie Polyethylen nach Aktivierung durch chemische Derivatisierung oder Plasmaaktivierung. Die Länge der Säulensegmente liegt zweckmäßigerweise im Bereich von 0,1µm bis 1mm, vorzugsweise 0,1 bis 100µm, höchstvorzugsweise 0,5 bis 10µm. Solche Schnitte lassen sich unschwer beispielsweise mittels eines Mikrotoms herstellen. Der Innendurchmesser der Säule liegt zweckmäßigerweise im Bereich von 0,05 bis 1 mm, vorzugsweise von 0,1 bis 0,5 mm, höchstvorzugsweise von 0,2 bis 0,4 mm.

Zweckmäßig ist es, wenn vor der eigentlichen Trennung (Bindung oder mechanische Auftrennung) der Nukleinsäuren unerwünschte Nukleinsäuren eliminiert werden. Unerwünschte Nukleinsäure sind beispielsweise Nukleinsäuren, welche an targetmolekülfreie Innenoberflächen der Säule binden. Dann kann eine targetmolekülfreie Säule vorgeschaltet und die Nukleinsäurebibliothek zuvor hierdurch geleitet werden. Unerwünschte Nukleinsäuren können solche sein die an bestimmte alternative Targetmoleküle nicht binden sollen, i.e. diskriminieren (beispielsweise nicht an natürliche Varianten onkogener Mutanten binden). Dann kann eine Säule mit den alternativen Targetmolekülen vorgeschaltet werden. Unerwünschte Nukleinsäuren können auch solche sein, welche an nur eine von mehreren Bindungsdomänen eines Targetmoleküls binden. Dann kann eine Säule mit Targetmolekülen, bei welchen eine oder mehrere Bindungsdomänen blockiert sind, vorgeschaltet sein.

Bei den vorstehenden Verfahrensweisen kann kontinuierlich, i.e. beispielsweise durch Hintereinanderschalten von Säulen, oder diskontinuierlich, beispielsweise durch zwischenzeitliches Auffangen des Eluenten aus der Vorsäule, gearbeitet werden.

Zur Erzielung einer weiteren Verbesserung der Affinitätstrennung kann grundsätzlich auf verschiedene Weisen verfahren werden. So können die aus einem oder mehreren Segmenten desorbierten Nukleinsäuren, ggf. nach Amplifikation wiederholt den erfindungsgemäßen Verfahren unterworfen werden, und/oder die Elutropie der Bedingungen bei der Desorption kann erhöht werden (Temperatur, Ionenstärke, pH-Wert, Pufferbedingungen). Alternativ kann die Raumdichte der Targetmoleküle und folglich der gebundenen Nukleinsäuren, i.e. die Anzahl der Targetmoleküle in einem Säulensegment, verringert werden.

Wenn mit einer homogenen Verteilung mehrer Targetmolekülspezies in einer Säule gearbeitet wird, so kann die Erfindung auf verschiedene Weisen weitergebildet sein. So ist es möglich, daß Nukleinsäuren gleicher oder ähnlicher Affinität, i.e. aus einem Säulensegment, nach der Desorption und Amplifikation nach Bindung an die verschiedenen Targetmolekülspezies getrennt werden durch Trennung der Targetmolekülspezies, beispielsweise durch Elektrophorese oder Chromatographie. Dabei kann die Bindung der Nukleinsäuren vor oder nach der Trennung der Targetmoleküle erfolgen. Im Rahmen dieser Nachtrennung gebundene Nukleinsäuren können dann auf alle in anderen Zusammenhängen vorstehend erläuterte Weisen von den Targetmolekülen gelöst bzw. desorbiert werden. Entsprechend kann gearbeitet werden, wenn ein Targetmolekül mehrere verschiedene Bindungsstellen aufweist und die Nukleinsäuren gegenüber dem Targetmolekül klein sind.

### Beispiel 1: Bestimmung der Empfindlichkeit einer Säule

Nimmt man ein quaderförmiges Targetmolekül der Dimension 100nm x 10nm x 10nm an, und nimmt man Bindung an das Strukturmaterial der Säule mit einer Hauptfläche an, so belegt ein Targetmolekül 10⁻¹⁵m². Ein Lumen von 1mm Durchmesser und 1 mm Länge weist eine Innenoberfläche von ca. 3x10⁻⁶ m² auf. Bei einem in der Säulentechnologie erfahrungsgemäß erhältlichen Belegungsgrad von 0,0001 bis 0,001 ergibt sich eine Anzahl von Targetmolekülen von 10⁵ - 10⁶ je mm Säule. Bei Schnitt der Säule in 1µm Säulensegmente mittels eines Mikrotoms ergibt sich folglich eine Anzahl von ca. 10² - 10³ Targetmolekülen je Säulensegment, womit eine extrem hohe Empfindlichkeit der Affinitätstrennung bei Einsatz üblicher Nukleinsäurebibliotheken erreichbar ist.

### Beispiel 2: Aktivierung einer Kieselgelsäule mit Tresylchlorid

Die Säule wird mit Aceton durchspült. Zur Aktivierung wird eine wasserfreie Lösung (2 ml Aceton, 1 ml Tresylchlorid, einige Tropfen Pyridin) durch die Säule gegeben (10faches Säulenvolumen) und diese über Nacht auf Eis inkubiert. Anschließend wird die Säule mit dem 20fachen Säulenvolumen 100 %Aceton (wasserfrei) durchspült. Die aktivierte Säule kann in 1 mM HCl aufbewahrt werden.

### Beispiel 3: Aktivierung einer Polyethylensäule

Ein Polyethylenschlauch wird mit dem 20fachen Säulenvolumen bei Raumtemperatur mit einer Lösung (2% Kaliumpermanganat (KMnO₄) (w/v) in konzentrierter Schwefelsäure (H2SO₄)) und anschließend mit destilliertem Wasser durchspült. Zur weiteren Kopplung der Säulenoberfläche können bi- oder polyvalente Moleküle zum Crosslinking verwendet werden, die mindestens eine reaktive Aldehydgruppe (z.B. 1% Glutaraldehyd ) aufweisen. Diese werden für 1 h bei 4°C durch die Säule gegeben. Anschließend wird die Reaktion durch reduzierende Bedingungen (z.B. durch Natrium Cyanoborhydrid (0.00025% w/v in 0.15 M NaCl, pH 3,9) stabilisiert.

### Beispiel 4: Koppelung eines Proteins an die aktivierte Kieselgelsäule.

Die Tresylchlorid- aktivierte Säule wird mit 0,1 M Na₂CO₃ (pH 8,5) durchspült. Zur Koppelung wird ein Peptid oder Protein (2mg/ml 0,1 M Na₂CO₃, pH 8,5) mehrfach für 2 h bei 37°C und anschließend für 4h auf Eis durch die Säule gegeben. Zur Blockierung freier Bindungstellen der Säule wird anschließend ein Überschuß von 0,2 M Glycin, pH 8 für durch die Säule gegeben.

### Beispiel 5: Koppelung eines Proteoglykans an die aktivierte Kieselgelsäule

Die Tresylchlorid- aktivierte Säule wird mit 0,1 M Na₂CO₃ (pH 8,5) durchspült. Zur Koppelung wird ein Peptid oder Protein (2mg/ml 0,1 M Na₂CO₃, pH 8,5) mehrfach über Nacht bei RT durch die Säule gegeben. Zur Blockierung freier Bindungstellen der Säule wird anschließend ein Überschuß von 0,2 M Glycin, pH 8 für durch die Säule gegeben.

### Beispiel 6: Koppelung eines Glykoproteins an die aktivierte Kieselgelsäule

Die Tresylchlorid- aktivierte Säule wird mit 0,1 M Na₂CO₃ (pH 8,5) durchspült. Zur Koppelung wird ein Peptid oder Protein (2mg/ml 0, 1 M Na₂CO₃, pH 8,5) mehrfach für 2 h bei 37°C und anschließend für 4h auf Eis durch die Säule gegeben. Zur Blockierung freier Bindungstellen der Säule wird anschließend ein Überschuß von 0,2 M Glycin, pH 8 durch die Säule gegeben.

### Beispiel 7: Koppelung eines Glykoproteins an die aktivierte Polyethylensäule

Die aktivierte Säule wird mit 0,1 M Na₂CO₃ (pH 8,5) durchspült. Zur Koppelung wird ein Peptid oder Protein (2mg/ml 0,1 M Na₂CO₃, pH 8,5) mehrfach für 2 h bei 37°C und anschließend für 4h auf Eis durch die Säule gegeben. Zur Blockierung freier Bindungstellen der Säule wird anschließend ein Überschuß von 0,2 M Glycin, pH 8 durch die Säule gegeben. Zur Verbesserung der Reaktion kann 1-Ethyl-3-(3-dimethylaminopropyl)carbodiimid (EDC), 5% w/v zugegeben werden).

### Beispiel 8: Herstellung einer Säule zur Eliminierung unerwünschter Moleküle

Die Tresylchlorid- aktivierte Säule wird mit 0,1 M Na₂CO₃ (pH 8,5) durchspült. Soll gegen ein Molekül mit einer oder mehreren primären oder sekundären Aminen die Elimination erfolgen wird dieses Molekül oder das Gemisch (2mg/ml 0,1 M Na₂CO₃, pH 8,5) mehrfach über Nacht bei RT durch die Säule gegeben. Zur Blockierung freier Bindungstellen der Säule wird anschließend ein Überschuß von 0,2 M Glycin, pH 8 für durch die Säule gegeben. Wird keine Elimination gegen bestimmte Moleküle mit einer oder mehreren primären oder sekundären Aminen gewünscht, werden alle Bindungstellen der Säule mit Glycin blockiert.

Weitere Derivatisierungsverfahren finden sich beispielsweise in den folgenden Literaturstellen: Patterson, W. J., National Aeronautics and Space Administration, Technical Memorandum, NASA TMX-73311, U.S. Government Printing Office, Washington, D.C., 1976, Ma, S. M., Gregonis, D. E., van Wagenen, R. A., and Andrade, J. D., in "Hydrogels for Medical and Related Applications" (J. D. Andrade, Ed.), Amer. Chem. Soc. Symp. Series, Vol. 31, p. 241, 1976, Harris, J. M., Struck, E. C., Case, M. G., Paley, M. S., Van Alstine, J. M., and Brooks, D. E., J. Polymer Sci., Polymer Chem. Ed., 22, 341 (1984), Regnier and Noel, Regnier, F. E., and Noel, R. J., J. Chromatog. Sci., 14, (1976), Yalpani, M. and Brooks, D. E., J. Polymer Sci., Polymer Chem. Ed., 23, 395 (1985).

### Beispiel 9: Durchführung des Kontaktes von Nukleinsäuren an die Targetmoleküle und Trennung der Säule

Die beschichteten Säulen werden Leckage frei hintereinander geschaltet, erst die Säulen zur Elimination unerwünschter Moleküle, anschließend die Säule mit den Targetmolekülen. Es ist möglich, auch Säulen mit jeweils verschiedenen Targetmolekülen leckagefrei hintereinander zu verknüpfen, da nach Entfernung von Nukleinsäuren mit unerwünschten Bindungseigenschaften aus dem Nukleinsäuregemisch und Entfernung von Nukleinsäuren mit Bindungsspezifität gegenüber einem oder mehreren Targetmolekülen aufgrund der kombinatorischen Zusammensetzung Nukleinsäuren gegen eine Vielzahl weiterer Targetmoleküle vorhanden sind. Zur Äquilibrierung wird ein geeigneter Puffer, z.B. eine Pufferlösung (10mM Tris-HCl, 50 mM KCl, 1,5 mM MgCl₂ und Gelatine 0,001% (w/v), pH8,3), für 1 h auf Eis über die Säule gegeben. Die Nukleinsäuren der kombinatorischen Nukleinsäurebibliothek werden in 1 ml des gleichen Puffers aufgenommen und zum Aufschmelzen von Doppelsträngen für 10 min auf 95°C erwärmt und anschließend mehrfach (4 - 30mal) über die Säule gegeben. Anschließend werden die Säulen getrennt und über Nacht auf Eis mit dem gewählten Puffer (s.o.) gewaschen. Die Auftrennung der Säule in Strömungsrichtung erfolgt mit einem geeigneten Schneidwerkzeug.

## Patentansprüche

1. Verfahren zur Selektion hochaffin an ein Targetmolekül bindender Nukleinsäuren aus einem Gemisch von Nukleinsäuren mit den folgenden Verfahrensschritten:
a) eine Säule wird innenseitig mit den Targetmolekülen beladen und die Targetmoleküle werden in der Säule immobilisiert,
b) das Gemisch an Nukleinsäuren wird einem ersten Ende der Säule aufgegeben, wobei durch die Säule ein definierter Volumenstrom an Trägersubstanz, laufend vom ersten Ende zum zweiten Ende der Säule, eingerichtet ist,
c) die Nukleinsäuren werden mit abnehmender Affinität der Nukleinsäuren zum Targetmolekül in zunehmendem Abstand von ersten Ende der Säule gebunden und immobilisiert,
d) der Volumenstrom an Trägersubstanz durch die Säule wird nach einer definierten Laufzeit beendet,
e) die Säule wird durch eine Mehrzahl von Teilungen in Säulensegmente zerschnitten, wobei jedem Segment eine Laufwegkoordinate zugeordnet wird,
f) aus zumindest einem Segment werden die immobilisierten Nukleinsäuren unspezifisch desorbiert und unter Zuordnung der dem Segment zugeordneten Laufwegkoordinate gewonnen.

2. Verfahren nach Anspruch 1, wobei in Verfahrensschritt f) die immobilisierten Nukleinsäuren aus jedem Segment separat desorbiert und gewonnen werden unter jeweiliger Zuordnung der Laufwegkoordinaten jeden Segments zu den daraus gewonnenen Nukleinsäuren.

3. Verfahren nach Anspruch 1 oder 2, wobei die unspezifische Desorption mittels physikochemischer oder thermischer Verfahren durchgeführt wird.

4. Verfahren nach einem der Ansprüche 1 bis 3, wobei die unspezifische Desorption durch thermische Desorption in einer, vorzugsweise verlängerten, Hochtemperaturphase einer Amplifikation, insbesondere PCR oder RT-PCR, durchgeführt wird.

5. Verfahren nach einem der Ansprüche 1 bis 4, wobei die unspezifische Desorption durch chemische Modifikation des Targetmoleküls und/oder durch Komplexbildung des Targetmoleküls mit einem Komplexbildner durchgeführt oder unterstützt wird.

6. Verfahren nach einem der Ansprüche 1 bis 5, wobei zwischen den Verfahrensschritten d) und e) zumindest eine Waschverfahrensschritt durchgeführt wird.

7. Verfahren nach einem der Ansprüche 1 bis 6, wobei die innenseitige Belegung der Säule mit Targetmolekülen mittels kovalenter Bindung, vorzugsweise über bifunktionale Spacerverbindungen, durchgeführt wird.

8. Verfahren nach einem der Ansprüche 1 bis 7, wobei jedes Säulensegment im statistischen Mittel 0,1 bis 10³, vorzugsweise 1 bis 10², höchstvorzugsweise 1 bis 10, Targetmoleküle enthält.

9. Verfahren nach einem der Ansprüche 1 bis 8, wobei als Strukturmaterial der Säule Kieselgel oder Polyethylen, vorzugsweise innenseitig der Säule chemisch derivatisiert und/oder plasmaaktiviert, verwendet wird.

10. Verfahren nach einem der Ansprüche 1 bis 9, wobei die Länge der Säulensegmente im Bereich von 0,1 µm bis 1 mm, vorzugsweise 0,1 bis 100 µm, höchstvorzugsweise 0,5 bis 10 µm, liegt.

11. Verfahren nach einem der Ansprüche 1 bis 10, wobei der Innendurchmesser der Säule im Bereich von 0,05 bis 1 mm, vorzugsweise 0,1 bis 0,5 mm, höchstvorzugsweise 0,2 bis 0,4 mm, liegt.

## Claims

1. A method for selecting nucleic acids that bond with high affinity to a target molecule from a mixture of nucleic acids, comprising the following steps:
a) a column is internally loaded with the target molecules and the target molecules are immobilized in said column,
b) the mixture of nucleic acids is fed to a first end of the column, wherein a defined volumetric flow of medium through the column is created, running from the first end to the second end of said column,
c) the nucleic acids are bond with an affinity to the target molecule that decreases as the distance from the first end of the column increases and are immobilized,
d) the volumetric flow of medium through the column is stopped after a defined period of time,
e) the column is divided into column segments by a plurality of partitions, a routing co-ordinate being allocated to each segment,
f) the immobilized nucleic acids from at least one segment are desorbed in a non-specific manner and are extracted wherein the routing co-ordinate allocated to the segment are allocated to the desorbed nucleic acids.

2. A method according to claim 1, wherein in step f) the immobilized nucleic acids from each segment are separately desorbed and obtained under respective allocation of the routing co-ordinate of each segment to the nucleic acids obtained therefrom.

3. A method according to claim 1 or 2, wherein the non-specific desorption is performed using physico-chemical or thermal methods.

4. A method according to one of claims 1 to 3, wherein the non-specific desorption is performed by thermal desorption in a preferably extended high-temperature phase of an amplification, in particular PCR or RT-PCR.

5. A method according to one of claims 1 to 4, wherein the non-specific desorption is performed or promoted by a chemical modification of the target molecule and/or by non-specific chelation with a chelating agent.

6. A method according to one of claims 1 to 5, wherein between steps d) and e) at least one washing step is performed.

7. A method according to one of claims 1 to 6, wherein the internal loading of the column with target molecules is performed by means of covalent binding, preferably by bifunctional spacer compounds.

8. A method according to one of claims 1 to 7, wherein each column segment contains in a statistical average 0,1 to 10³, preferably 1 to 10², most preferably 1 to 10 target molecules.

9. A method according to one of claims 1 to 8, wherein as the constructional material of the column, silica gel or polyethylene, preferably derivatized and/or plasma-activated on the inner side of the column, is used.

10. A method according to one of claims 1 to 9, wherein the length of the column segments is in the range from 0, 1 µm to 1 mm, preferably 0, 1 to 100 µm, most preferably 0,5 to 10 µm.

11. A method according to one of claims 1 to 10, wherein the inner diameter of the column is in the range from 0,05 to 1 mm, preferably 0, 1 to 0,5 mm, most preferably 0,2 to 0,4 mm.

## Revendications

1. Procédé pour sélectionner des acides nucléiques se liant avec une haute affinité à une molécule cible à partir d'un mélange d'acides nucléiques, consistant en les étapes suivantes:
a) une colonne est chargée à son côté intérieur avec les molécules cibles, et les molécules cibles sont immobilisées dans la colonne,
b) le mélange d'acides nucléiques est alimenté à une première extrémité de la colonne, un débit de volume défini de substance support étant effectué à travers la colonne et courant à partir de la première extrémité à la deuxième extrémité de la colonne,
c) les acides nucléiques sont liés et immobilisés selon l'affinité décroissante des acides nucléiques à la molécule cible en une distance croissante de la première extrémité de la colonne,
d) le débit de volume de substance support à travers la colonne est terminé après un temps défini,
e) la colonne est divisée par une multiplicité de divisions en des segments de colonne, à chaque segment une coordonnée de trajet étant associée,
f) à partir d'au moins un segment, les acides nucléiques immobilisés sont désorbés de manière non spécifique et sont obtenus sous association de la coordonnée de trajet associée à ce segment.

2. Procédé selon la revendication 1, dans lequel dans l'étape f) les acides nucléiques immobilisés sont désorbés séparément de chaque segment et sont obtenus sous association respective des coordonnées de trajet de chaque segment aux acides nucléiques obtenus.

3. Procédé selon la revendication 1 ou 2, dans lequel la désorption non spécifique est exécutée au moyen de procédés physico-chimiques ou thermiques.

4. Procédé selon une des revendications 1 à 3, dans lequel la désorption non spécifique est exécutée par désorption thermique dans une phase à haute température, de préférence prolongée, d'une amplification, en particulier PCR ou RT-PCR.

5. Procédé selon une des revendications 1 à 4, dans lequel la désorption non spécifique est exécutée ou soutenue par modification chimique de la molécule cible et/ou complexation de la molécule cible avec un agent complexant.

6. Procédé selon une des revendications 1 à 5, dans lequel entre les étapes d) et e) au moins une étape de lavage est exécutée.

7. Procédé selon une des revendications 1 à 6, dans lequel l'application de molécules cibles sur le côté intérieur de la colonne est exécutée au moyen de liage covalent, de préférence par l'intermédiaire de composés d'espacement bifonctionnels.

8. Procédé selon une des revendications 1 à 7, dans lequel chaque segment de la colonne contient au moyen statistique 0,1 à 10³, de préférence 1 à 10², de préférence la plus haute 1 à 10 molécules cibles.

9. Procédé selon une des revendications 1 à 8, dans lequel comme matériau structurel de la colonne, du gel de silice ou du polyéthylène est utilisé, de préférence dérivatisé chimiquement et/ou activé par plasma sur le côté intérieur de la colonne.

10. Procédé selon une des revendications 1 à 9, dans lequel la longueur des segments de colonne est comprise dans la gamme entre 0,1 µm et 1 mm, de préférence 0,1 et 100 µm, de préférence la plus haute 0,5 et 10 µm.

11. Procédé selon une des revendications 1 à 10, dans lequel le diamètre intérieur de la colonne est compris dans la gamme entre 0,05 et 1 mm, de préférence 0,1 et 0,5 mm, de préférence la plus haute 0,2 et 0,4 mm.
